(19) Europäisches Patentamt
European Patent Office
Office européen des brevets

(11) EP 1 087 924 B1

(12) **FASCICULE DE BREVET EUROPEEN**

(45) Date de publication et mention de la délivrance du brevet:
**25.02.2004 Bulletin 2004/09**

(21) Numéro de dépôt: **99925110.1**

(22) Date de dépôt: **16.06.1999**

(51) Int Cl.[7]: **C07C 51/367**, C07C 59/64, C07C 59/52, C07C 51/377, C07C 45/67

(86) Numéro de dépôt international:
**PCT/FR1999/001442**

(87) Numéro de publication internationale:
**WO 1999/065853 (23.12.1999 Gazette 1999/51)**

(54) **PROCEDE DE PREPARATION DE COMPOSES P-HYDROXYMANDELIQUES EVENTUELLEMENT SUBSTITUES**

VERFAHREN ZUR HERSTELLUNG VON GEGEBENENFALLS SUBSTITUIERTEN P-HYDROXYMANDELSÄUREN

METHOD FOR PREPARING P-HYDROXYMANDELIC COMPOUNDS OPTIONALLY SUBSTITUTED

(84) Etats contractants désignés:
**AT BE CH DE DK ES FI FR GB IT LI NL SE**

(30) Priorité: **16.06.1998 FR 9807586**

(43) Date de publication de la demande:
**04.04.2001 Bulletin 2001/14**

(73) Titulaire: **RHODIA CHIMIE**
**92408 Courbevoie Cédex (FR)**

(72) Inventeurs:
- **JOUVE, Isabelle**
**F-69740 Genas (FR)**
- **FOURNET, Frédéric**
**F-69270 Couzon au Mont d'Or (FR)**
- **FRAGNON, Jean**
**F-69330 Meyzieu (FR)**

(74) Mandataire: **Dutruc-Rosset, Marie-Claude et al**
**Rhodia Services,**
**Direction de la Propriété Industrielle,**
**40, rue de la Haie-Coq**
**93306 Aubervilliers Cedex (FR)**

(56) Documents cités:
**EP-A- 0 578 550**
**US-A- 4 337 355**
**US-A- 4 401 830**
**US-A- 5 248 816**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 087 924 B1

## Description

La présente invention a pour objet un procédé de préparation de composés p-hydroxymandéliques éventuellement substitués et dérivés.

Dans l'exposé qui suit de l'invention, on entend par "composés p-hydroxymandéliques éventuellement substitués" un composé, aromatique au moins porteur d'un groupe -CHOH-COOH en position para d'un groupe hydroxyle.

La présente invention vise plus particulièrement la préparation de l'acide p-hydroxymandélique et de l'acide méthoxy-3 p-hydroxymandélique.

L'une des voies de synthèse classique des acides p-hydroxymandéliques consiste à effectuer la condensation en milieu alcalin, de l'acide glyoxylique sur le phénol et/ou ses dérivés correspondants.

Le rendement est limité par le fait que la réaction de condensation n'est pas sélective et conduit également aux acides o-hydroxymandéliques et aux acides dimandéliques.

De plus, le rendement réactionnel est diminué en raison d'une réaction secondaire parasite. En effet, l'acide glyoxylique en milieu alcalin aqueux, est transformé selon la réaction de Cannizaro, en acides oxalique et glycolique.

Pour éviter que cette réaction de Cannizaro devienne prépondérante et détruise l'acide glyoxylique, on a proposé selon FR-A 2 132 364 de conduire la réaction de condensation, en milieu aqueux dilué et à basse température ou température ambiante.

Compte-tenu de la difficulté d'obtenir des rendements, réactionnels satisfaisants, il importe de contrôler les différents paramètres de procédé et, en particulier, la qualité de l'acide glyoxylique engagé.

Le procédé le plus important d'un point de vue industriel de préparation de l'acide glyoxylique, consiste à oxyder le glyoxal par l'acide nitrique. On obtient ainsi des solutions aqueuses d'acide glyoxylique, qui, outre le glyoxal n'ayant pas réagi, contiennent également de l'acide oxalique, des acides organiques, tels que l'acide formique, l'acide acétique, l'acide glycolique et de l'acide nitrique.

Jusqu'à présent, on n'a pas cessé de chercher de nouvelles méthodes de séparation et de purification de l'acide glyoxylique.

C'est ainsi que l'on a proposé dans DE-A 1 198 339, un procédé qui permet d'éliminer tout d'abord l'acide nitrique, puis l'acide oxalique à l'aide de résines échangeuses d'ions basiques, puis le glyoxal et les autres impuretés par surconcentration de la solution et cristallisation.

On a divulgué dans DE-A 2 501 743, un procédé selon lequel l'acide glyoxylique est séparé de ces impuretés par extraction à l'aide d'alcools aliphatiques ou cyctoatiphatiques ou d'esters d'alcools aliphatiques à faible condensation en carbone.

On a également décrit dans FR-A 2 552 426, un procédé d'obtention de solutions aqueuses d'acide glyoxylique exemptes d'autres acides qui consiste à traiter la solution de départ par un composé azoté organique, de préférence une amine tertiaire à une température au plus égale à 50°C puis à extraire l'acide glyoxylique par épuisement de la phase organique avec de l'eau, à une température plus élevée.

On note donc dans l'état de la technique, un souci constant de fournir une solution d'acide glyoxylique débarrassée de ses impuretés.

Allant à l'encontre de cet enseignement, on a trouvé que, dans le cadre de la préparation des composés p-hydroxymandéliques éventuellement substitués, la condensation de l'acide glyoxylique et du phénol corespondant est effectuée avec un rendement accru dès lors que ladite réaction est conduite en présence d'un acide dicarboxylique mis en oeuvre en une certaine quantité.

La présente invention a précisément pour objet un procédé de préparation de composés p-hydroxymandéliques éventuellement substitués et dérivés qui consiste à effectuer la condensation dans l'eau, en présence d'un agent alcalin, d'un composé aromatique porteur d'au moins un groupe hydroxyle et dont la position en para est libre, avec l'acide glyoxylique, ledit procédé étant caractérisé par le fait que la réaction est conduite en présence d'une quantité efficace d'un composé porteur d'au moins deux fonctions carboxyliques.

Conformément au procédé de l'invention, la mise en oeuvre du catalyseur de l'invention permet d'accroître le rendement réactionnel.

Un autre avantage du procédé de l'invention est qu'il peut faire appel à un acide glyoxylique plus technique et contenant entre autres de l'acide oxalique.

Le procédé de l'invention s'applique tout particulièrement au phénol mais aussi aux phénols substitués ayant au moins une position en para- non substituée.

Le noyau aromatique est porteur d'au moins un groupe hydroxyle mais il peut être également porteur d'un ou plusieurs autres substituants. Généralement, par plusieurs substituants, on définit moins de quatre substituants par noyau aromatique.

N'importe quel substituant peut être présent dans la mesure où il n'interfère pas dans la réaction de l'invention.

Ainsi, le procédé de l'invention est bien adapté pour s'appliquer aux composés aromatiques hydroxylés répondant à la formule suivante (I) :

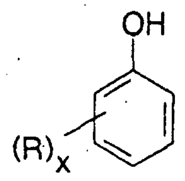

(I)

dans ladite formule (I) :

- la position en para est libre,
- x est un nombre entier compris entre 1 et 4,
- R représente:
  - . un atome d'hydrogène,
  - . un groupe hydrocarboné ayant de 1 à 20 atomes de carbone choisi parmi
  - . les groupes alkyle, alkoxy, hydroxyalkyle, cycloalkyle, aryle, phénoxy, alkoxyalkyle, fluoroalkyle, hydroxyalkoxyalkylène,
  - . un groupe hydroxyle,
  - . un groupe -CHO,
  - . un groupe acyle ayant de 2 à 6 atomes de carbone,
  - . un atome d'halogène, de préférence un atome de fluor, de chlore ou de brome.
  - . deux groupes R placés sur deux atomes de carbone vicinaux peuvent former ensemble et avec les atomes de carbone qui les portent un cycle benzénique.

On donne ci-après des exemples de radicaux R susceptibles d'être portés par le noyau aromatique :

- radicaux alkyle tels que méthyle, éthyle, propyle, isopropyle, butyle, isobutyle, sec-butyle, tert-butyle, n-octyle, éthyl-2 hexyle, décyle, octadécyle, eicosyle,
- radicaux alkoxy tels que méthoxy, éthoxy, propoxy; isopropoxy, butoxy, isobutoxy, sec-butoxy, tert-butoxy, hexyloxy, décyloxy, hexadécyloxy, octadécyloxy ou un radical phénoxy,
- radicaux hydroxyalkyle tels que hydroxyméthyle, hydroxyéthyle, hydroxypropyle, hydroxyhexyle, hydroxydécyle,
- radicaux cycloalkyle tels que cyclopentyle, cyclohexyle, cycloheptyle,
- radicaux fluoroalkyle tels que fluorométhyle, difluorométhyle, trifluorométhyle, fluoroéthyle, triftuoro-1,1,1 éthyle, pentafluoroéthyle, fluoropropyle, fluorobutyle, trifluoroamyle,
- radicaux hydroxyalkyoxyalkylène tels que hydroxyméthyloxyéthylène, hydroxyéthyl di-(oxyéthylène), hydroxyéthyl tri-(oxyéthylène), hydroxyéthyloxypropylène-1,2, hydroxyéthyloxybutylène, hydroxypropyloxypropylène, hydroxybutyloxybutylène, hydroxybutyl di-(oxybutylène),
- atomes d'halogène tels que fluor, chlore, brome ou iode.

On met en oeuvre tout préférentiellement dans le procédé de l'invention, les composés aromatiques hydroxylés répondant à la formule générale (I) dans laquelle :

- x est égal à 0, 1, 2 ou 3,
- R représente l'un des groupes ou fonctions suivantes :
  - . un atome d'hydrogène,
  - . un radical alkyle, linéaire ou ramifié, ayant de 1 à 10 atomes de carbone, de préférence de 1 à 4 atomes de carbone,
  - . un radical alkoxy linéaire ou ramifié ayant de 1 à 10 atomes de carbone, de préférence de 1 à 4 atomes de carbone,
  - . un groupe - OH,
  - . un groupe - CHO,
  - . un atome d'halogène.
  - . un groupe - $CF_3$

Encore plus préférentiellement, on choisit les composés de formule (I) dans laquelle les radicaux R identiques ou différents sont un atome d'hydrogène, un radical alkyle, linéaire ou ramifié ayant de 1 à 4 atomes de carbone tels

que les radicaux méthyle, éthyle, n-propyle, isopropyle, n-butyle ou isobutyle, un radical alkoxy linéaire ou ramifié ayant de 1 à 4 atomes de carbone comme les radicaux méthoxy ou éthoxy, un groupe - CHO ou un atome de chlore et x est de préférence égal à 0 ou 1.

A titre illustratif de composés répondant à la formule (I), on peut mentionner :

- ceux répondant à la formule (I) dans laquelle x est égal à 0, tels que le phénol,
- ceux répondant à la formule (I) dans laquelle x est égal à 1, tels que :
  - . la pyrocatéchine
  - . la résorcine
  - . l'o-crésol
  - . le m-crésol
  - . l'éthyl-2 phénol
  - . l'éthyl-3 phénol
  - . le propyl-2 phénol
  - . le sec-butyl-2 phénol
  - . le tert-butyl-2 phénol
  - . le tert-butyl-3 phénol
  - . le méthoxy-2 phénol (gaïacol)
  - . le méthoxy-3 phénol
  - . l'éthoxy-2 phénol (guétol)
  - . l'isopropoxy-2 phénol
  - . l'aldéhyde salicylique
  - . le salicylate de méthyle
  - . le chloro-2 phénol
  - . le chloro-3 phénol
  - . le nitro-3 phénol
- ceux répondant à la formule (I) dans laquelle x est égal à 2, tels que :
  - . le diméthyl-2,3 phénol
  - . le diméthyl-2,5 phénol
  - . le diméthyl-3,5 phénol
  - . l'hydroxy-2 acétamido-5 benzaldéhyde
  - . l'hydroxy-2 éthamido-5 benzaldéhyde
  - . le dichloro-2,3 phénol
  - . le dichloro-2,5 phénol
  - . le dichloro-3,5 phénol
  - . le pyrogallol
- ceux répondant à la formule (I) dans laquelle x est égal à 3, tels que :
  - . le triméthyl 2,3,5 phénol
  - . le di-tert butyl-3,5 phénol
  - . le trichloro-2,3,5 phénol
- ceux répondant à la formule (I) présentant un radical naphtalénique, tels que :
  - . le naphtol-1
  - . le naphtol-2
  - . le dihydroxy-1,2 naphtalène
  - . le dihydroxy-1,5 naphtalène
  - . le dihydroxy-2,3 naphtalène
  - . le dihydroxy-2,6 naphtalène
  - . le dihydroxy-2,7 naphtalène
  - . le bromo-6 naphtol-2
- ceux répondant à la formule (I) présentant un enchaînement de noyaux benzéniques:

- le phénoxy-2 phénol
- le phénoxy-3 phénol

Parmi la liste des composés précités, les composés aromatiques porteurs d'au moins un groupe hydroxyle mis en oeuvre préférentiellement sont : le phénol, l'o-crésol, le m-crésol, l'éthyl-3 phénol, le tert-butyl-2 phénol, le gaïacol, le guétol.

En ce qui concerne la nature du catalyseur mis en oeuvre, on peut faire appel à un acide au moins difonctionnel répondant à la formule (II) suivante :

$$HOOC - R_1 - COOH \qquad (II)$$

dans ladite formule (II), $R_1$ représente un lien valentiel ou un radical hydrocarboné éventuellement substitué comportant de 1 à 40 atomes de carbone.

Plus précisément, dans la formule (II), $R_1$ symbolise un radical hydrocarboné, substitué ou non qui peut être un radical aliphatique acyclique, saturé ou insaturé, linéaire ou ramifié ; un radical carbocyclique, saturé, insaturé ou aromatique, monocyclique ou polycyclique ; un radical hétérocyclique, saturé, insaturé ou aromatique, monocyclique ou polycyclique.

Conviennent tout particulièrement bien à la mise en oeuvre du procédé de l'invention, les composés porteurs d'au moins deux fonctions carboxyliques de formule générale (II) dans laquelle $R_1$ représente un lien valentiel ou un radical divalent ayant de préférence de 1 à 15 atomes de carbone.

Sont particulièrement bien adaptés à la mise en oeuvre du procédé de l'invention, les composés porteurs d'au moins deux fonctions carboxyliques de formule générale (II) dans laquelle $R_1$ représente un reste aliphatique acyclique, saturé ou insaturé, linéaire ou ramifié.

Plus précisément, $R_1$ représente un reste aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

La chaîne hydrocarbonée peut être éventuellement :

(1) - interrompue par l'un des groupes suivants dénommés Y :

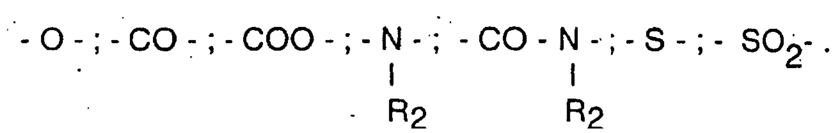

dans ces formules $R_2$ représente l'hydrogène ou un radical alkyle linéaire ou ramifié ayant de 1 à 4 atomes de carbone, de préférence, un radical méthyle ou éthyle ou un radical de type $-(CH_2)_p$ - COOH dans lequel p est un nombre compris entre 1 et 5,

(2) - et/ou porteuse de l'un des substituants suivants :

- OH; -COOH; -CHO; $-NO_2$; -CN; $-NH_2$; -SH; -X; $-CF_3$.
- $NH-[(CH_2)_p-COOH]$ ou - $N-[(CH_2)_p - COOH]_2$

avec X représentant un atome d'halogène, de préférence un atome de . fluor, de chlore ou de brome et p ayant la signification donnée précédemment.

Conviennent également à la mise en oeuvre du procédé de l'invention, les composés porteurs d'au moins deux fonctions carboxyliques de formule générale (II) dans laquelle $R_1$ représente un reste hydrocarboné aromatique monocyclique ou polycyclique.

$R_1$ représente préférentiellement un reste hydrocarboné aromatique, et notamment benzénique répondant à la formule générale (III) :

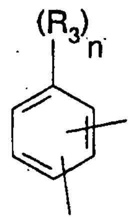

(III)

dans ladite formule (III):

- n est un nombre entier de 0 à 4, de préférence de 0 à 3,
- $R_3$ représente l'un des groupes ou fonctions suivantes :
  - . un atome d'hydrogène,
  - . un radical alkyle linéaire ou ramifié, ayant de à 4 atomes de carbone,
  - . un radical alkoxy linéaire ou ramifié, ayant de 1 à 4 atomes de carbone,
  - . un radical méthylène ou éthylène dioxy,
  - . un groupe -CHO,
  - . un radical phényle ou benzyle,
  - . un atome d'halogène.

Encore plus préférentiellement, on choisit les composés de formule (II) dans laquelle le radical $R_1$ répond à la formule (III) dans laquelle les radicaux $R_3$ identiques ou différents sont un atome d'hydrogène, un radical méthyle, un radical méthoxy, un groupe - CHO.

Les composés porteurs d'au moins deux fonctions carboxyliques peuvent répondre à la formule générale (II) dans laquelle le radical $R_1$ représente un reste divalent hydrocarboné aromatique polycyclique ; les cycles pouvant former entre eux des systèmes ortho condensés, ortho- et péri-condensés. On peut citer plus particulièrement, un reste naphtylénique ; lesdits cycles pouvant être substitués par 1 à 4 radicaux $R_3$ de préférence 1 à 3, $R_3$ ayant les significations énoncées précédemment pour les substituants du reste hydrocarboné aromatique de formule générale (III).

Dans là formule générale (II) des composés porteurs d'au moins deux fonctions carboxyliques, $R_1$ peut représenter également un reste carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux $R_3$ de préférence 1 à 3, $R_3$ ayant les significations énoncées précédemment pour les substituants du reste hydrocarboné aromatique de formule générale (III).

Comme exemples préférés de radicaux $R_1$, on peut citer les radicaux cyclohexane-diyle, éventuellement substitué par des radicaux alkyle linéaires ou ramifiés, ayant de 1 à 4 atomes de carbone.

Les composés porteurs d'au moins deux fonctions carboxyliques peuvent également répondent à la formule (II) dans laquelle $R_1$ représente un radical divalent constitué par un enchaînement de deux à quatre restes tels que définis précédemment, resté aliphatique, reste aromatique ou cycloaliphatique. Ceux-ci peuvent être reliés entre eux par un lien valentiel ou par un groupe fonctionnel qui peut être notamment un groupe choisi parmi les groupes dénommés Y.

On donne ci-après quelques exemples de radicaux $R_1$ :

- $CH_2$-$C_6H_4$-;
- $CH_2$-$CH_2$-$C_6H_4$-;
- $CH_2$-O-$C_6H_4$-;
- $CH_2$-O-$C_6H_4$-;
- $CH_2$-O-$C_6H_4$-$CH_2$-
- $C_6H_4$-$C_6H_4$-
- $C_6H_4$-$CH_2$-$C_6H_4$-
- $C_6H_4$-O-$C_6H_4$-
- $CH_2$-$C_6H_4$-$CH_2$-$C_6H_4$-$CH_2$-

A titre de catalyseurs convenant à la présente invention, on peut citer tout particulièrement les composés porteurs d'au moins deux fonctions carboxyliques suivants :

- les acides aliphatiques dicarboxyliques tels que :

  - l'acide oxalique
  - l'acide malonique.
  - l'acide succinique
  - l'acide glutarique
  - l'acide adipique
  - l'acide diméthyl-2,4 adipique
  - l'acide pimélique
  - l'acide subérique
  - l'acide azélaïque
  - l'acide sébacique
  - l'acide dodécane dioïque
  - l'acide fumarique
  - l'acide maléique

- les acides cycloalcanedicarboxyliques tels que l'acide cyclohexane dicarboxylique-1,4,
- les acides aromatiques dicarboxyliques tels que :

  - l'acide phtalique
  - l'acide isophtalique
  - l'acide téréphtalique
  - l'acide phénylènediacétique
  - l'acide naphtalène dicarboxylique-1,5
  - l'acide naphtalène dicarboxylique-1,6
  - l'acide diphénylcarboxylique-4,4'
  - l'acide diphénylcarboxylique-3,3'
  - l'oxyde de bis(hydroxycarbonyl-4) phényle
  - l'oxyde de bis(hydroxycarbonyl-3)phényle
  - la dihydroxycarbonyl-4,4' diphénylsulfone
  - la dihydroxycarbonyl-3,3' diphénylsulfone

- les acides pyrimidines ou imidazoles dicarboxyliques.

Dans la liste des acides dicarboxyliques précités, les composés mis en oeuvre préférentiellement sont : l'acide oxalique, l'acide malonique, l'acide succinique, l'acide glutarique, l'acide adipique, l'acide sébacique, l'acide phtalique, l'acide isophtalique, l'acide téréphtalique.

Sont également parfaitement bien adaptés à la mise en oeuvre du procédé de l'invention, les acides aminopolycarboxyliques. Comme exemples d'acides aminopolycarboxyliques susceptibles d'être mis en oeuvre dans le procédé de l'invention, on peut mentionner entre autres :

- l'acide éthylènediaminotétracétique (E.D.T.A.)
- l'acide diéthylènetriaminopentacétique (D.T.P.A.)
- l'acide nitrilotriacétique (N.T.A.)
- l'acide N-(2-hydroxyéthyl)éthylène diaminotriacétique (H.E.D.T.A.)

Parmi les acides aminopolycarboxyliques précités, on choisit préférentiellement, l'acide éthylènediaminotétracétique.

Selon le procédé de l'invention, la réaction est conduite en présence d'un hydroxyde de métal alcalin qui peut être l'hydroxyde de sodium ou de potassium.

Pour des considérations économiques, on choisit préférentiellement l'hydroxyde de sodium.

En ce qui concerne les concentrations et les quantités de réactifs à mettre en oeuvre, on définit ci-après, les conditions préférées.

Conformément au procédé de l'invention, on fait appel à une solution d'acide glyoxylique. La concentration de ladite solution n'est pas critique et peut varier largement, par exemple, entre 15 et 70 % en poids. On a recours, d'une manière préférée, aux solutions commerciales dont la concentration est d'environ 50 %.

Selon le procédé de l'invention, on fait réagir l'acide glyoxylique sur le composé aromatique hydroxylé de formule (I) en excès. Le rapport molaire entre le composé aromatique hydroxylé de formule (I) et l'acide glyoxylique

varie entre 1,5 et 4,0 et est choisi préférentiellement entre 2,0 et 3,0.

La solution d'hydroxyde de métal alcalin mise en oeuvre a une concentration généralement comprise entre 10 et 50 % en poids. La concentration de la solution de départ n'est pas critique. Toutefois, comme la concentration du composé aromatique hydroxylé de formule (I) est avantageusement faible dans le milieu réactionnel, on utilise une solution diluée de métal alcalin pour effectuer la dilution du milieu réactionnel.

La quantité d'hydroxyde de métal alcalin introduite dans le milieu réactionnel tient compte de la quantité nécessaire pour salifier la fonction hydroxyle du composé aromatique hydroxylé de formule (I) et de la quantité nécessaire pour salifier la fonction carboxylique de l'acide glyoxylique.

Si le composé aromatique hydroxylé de formule (I) présente des fonctions salifiables autres que le groupe hydroxyle, on introduit donc la quantité d'hydroxyde de métal alcalin nécessaire pour salifier toutes les fonctions salifiables qui peuvent être des groupes hydroxyle et/ou des fonctions carboxylique COOH.

Généralement, la quantité d'hydroxyde de métal alcalin peut varier largement et être égale ou voisine de la stoechiométrie ou en excès.

Généralement, la quantité d'hydroxyde de métal alcalin varie entre 80 et 120 % de la quantité stoechiométrique.

La concentration du composé aromatique hydroxylé de formule (I) est comprise de préférence entre 0,5 et 1,5 moles/litre, et plus particulièrement aux environs de 1 mole/litre.

En ce qui concerne la quantité de catalyseur mise en oeuvre, celle-ci est déterminée de telle sorte que le rapport molaire entre le catalyseur et le composé aromatique hydroxylé de formule (I) se situe entre 0,005 et 0,025 et de préférence entre 0,01 et 0,02.

La quantité de catalyseur mise en oeuvre exprimée par le rapport entre le nombre de moles de catalyseur et le nombre de moles d'acide glyoxylique est choisie avantageusement entre 0,5 et 2,5 % de préférence, entre 1 et 2 %.

Le catalyseur préféré est l'acide oxalique.

Les solutions commerciales d'acide glyoxylique peuvent contenir de très faibles quantités d'acide oxalique. L'acide oxalique de la réaction peut donc être fourni en partie par la solution de départ. Dans ce cas; il y aura lieu de compléter la quantité d'acide oxalique par addition d'acide oxalique, ou de tout autre acide dicarboxylique de telle sorte que l'on respecte les rapports précités.

Selon un mode de réalisation préférée de l'invention, on met en oeuvre avantageusement une solution d'acide glyoxylique comprenant de 0,6 à 3 %, de préférence, de 1,2 à 2,6 % en poids d'acide oxalique exprimé par rapport au poids d'acide glyoxylique.

La température de la réaction est choisie avantageusement entre 20°C et 60°C, et de préférence entre 30°C et 40°C.

Le procédé de l'invention est conduit à pression atmosphérique mais sous atmosphère contrôlée de gaz inertes, de préférence d'azote ou de gaz rares, en particulier d'azote.

On donne ci-après un mode préféré de réalisation pratique de l'invention.

Dans un milieu réactionnel comprenant le composé aromatique hydroxylé de formule (I), de l'eau et de l'hydroxyde de métal alcalin en quantité nécessaire pour salifier le groupe hydroxyle et d'autres éventuelles fonctions salifiables du composé de formule (I), on introduit la solution d'acide glyoxylique et le catalyseur et en parallèle la solution d'hydroxyde de métal alcalin mis en oeuvre en une quantité nécessaire pour salifier la fonction COOH.

On maintient le milieu réactionnel sous agitation et à la température choisie dans l'intervalle précité pendant une durée variable allant de 1 à 10 heures.

Une autre variante d'exécution de l'invention consiste à rajouter le catalyseur de la réaction non pas dans la solution aqueuse d'acide glyoxylique, mais simultanément avec le composé aromatique hydroxylé de formule (I).

En fin de réaction, on sépare l'acide p-hydroxymandélique éventuellement substitué obtenu sous forme salifiée selon les techniques classiques de séparation, notamment par cristallisation.

Le procédé de l'invention s'applique tout particulièrement bien lorsque l'on fait appel à une solution aqueuse d'acide glyoxylique comprenant des acides monofonctionnels tels que l'acide acétique, formique et glycolique et notamment lorsqu'il y a présence d'acide acétique dont la concentration varie entre 0,1 et 3%.

Le procédé de l'invention conduit à l'obtention de composés p-hydroxymandéliques éventuellement substitués qui peuvent être représentés par la formule (IV) suivante :

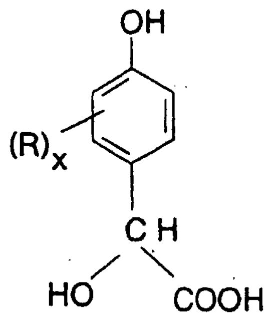

(IV)

dans ladite formule (IV), R et x ayant la signification donnée dans la formule (I).

Ces produits sont particulièrement intéressants car ce sont des produits intermédiaires permettant entre autres, d'obtenir par réduction, des acides hydroxyarylacétiqûes ou par oxydation, des acides hydroxyarylglyoxyliques (= hydroxyaryl α-oxo acétiques) ou des aldéhydes hydroxyaromatiques.

Une application préférée de l'invention est la préparation d'aldéhydes hydroxyaromatiques, par oxydation des composés de formule (IV) obtenus selon l'invention.

L'oxydation des composés de formule (IV) peut être conduite selon les techniques décrites dans la littérature. Ainsi, on peut se référer à P. HEBERT [Bull. Soc. Chim. France, 27, p.45-55(1920)] et à NAGAI SHIGEKI et al, [JP-A 76/128934]. L'oxydation est généralement conduite par l'oxygène ou l'air sous pression, en présence d'un catalyseur approprié tel que par exemple, les dérivés du chrome, cobalt , cuivre, vanadium ou osmium.

Ainsi, l'invention permet d'accéder facilement à l'hydroxy-4 benzaldéhyde et à la vanilline et ses analogues, par exemple éthyl-3, isopropyl-3 vanilline, par oxydation respectivement de l'acide p-hydroxymandélique et des acides méthoxy-3 p-hydroxymandélique, éthoxy-3 p-hydroxy-mandélique, ou isopropoxy-3 p-hydroxymandélique.

Les exemples qui suivent illustrent l'invention sans toutefois la limiter.

Dans les exemples, les pourcentages indiqués sont exprimés en poids.

Les abréviations mentionnées dans les exemples ont la signification suivante :

$$\text{Conversion (TT)} = \frac{\text{nombre de moles de gaïacol transformées}}{\text{nombre de moles de gaïacol introduites}}$$

$$\text{Rendement (R R)} = \frac{\text{nombre de moles d'acide mandélique formées}}{\text{nombre de moles d'acide glyoxylique introduites}}$$

$$\text{Sélectivité (RT)} = \frac{\text{nombre de moles d'acide mandélique formées}}{\text{nombre de moles de gaïacol transformées}}$$

Exemple 1

Dans un réacteur en verre d'un litre muni d'une double-enveloppe, d'une électrode de pH, d'une sonde de température, d'un réfrigérant, d'une arrivée de gaz inerte et d'une agitation mécanique, on charge :

- 600 g d'eau distillée,
- 91,6 g .(0,687 mol) d'une solution aqueuse de soude à 30 %,
- 93 g (0,750 mol) de gaïacol

On établit l'atmosphère inerte et l'on porte le mélange réactionnel à 35°C et l'on ajoute simultanément en 2 heures, 50,7 g (0,380 mol) d'une solution aqueuse de soude à 30 % et 55,2 g d'une solution aqueuse d'acide glyoxylique à 50 % en poids. On ajoute avec l'acide glyoxylique, de l'acide oxalique mis en jeu en une quantité telle qu'il représente 0,75 % en poids de la solution d'acide glyoxylique.

La solution d'acide glyoxylique mise en jeu contient de l'acide oxalique à raison de 0,3 %, des acides carboxyliques inférieurs tels que l'acide acétique à raison de 0,9 % , de l'acide formique et glycolique en une quantité respective inférieure à 0,1 %.

On maintient le mélange réactionnel à 35°C pendant 2 heures.

En fin de réaction, on dose les produits de la réaction par chromatographie liquide haute performance.

Les résultats obtenus sont les suivants :

- conversion :
  - . TT = 47,3%
- acide hydroxy-4 méthoxy-3 mandélique :
  - . RR = 79,7 %
  - . RT = 84,2 %
- acide hydroxy-2 méthoxy-3 mandélique :
  - . RR = 4,8 %
  - . RT = 5,1 %
- acide hydroxy-2 méthoxy-3 dimandélique-1,5 :
  - . RR = 8,0%
  - . RT = 4,0 %.

Exemple comparatif 2

On reproduit l'exemple 1 à la différence que l'on ne charge pas d'acide oxalique.
Les résultats obtenus sont les suivants:

- conversion ;
  - . TT = 46,1 %
- acide hydroxy-4 méthoxy-3 mandélique :
  - . RR = 76,9 %
  - . RT = 83,0 %
- acide hydroxy-2 méthoxy-3 mandélique :
  - . RR = 5,1 %
  - . RT = 5,5 %
- acide hydroxy-2 méthoxy-3 dimandélique-1,5 :
  - . RR = 7,5 %
  - . RT = 4,1 %

Exemple 3

Dans cet exemple, on reproduit l'exemple 1 mais en mettant en oeuvre une solution d'acide glyoxylique à 50% % contenant 0,4 % en poids d'acide oxalique.
Les résultats obtenus sont les suivants :

- conversion;
  - . TT = 48%
- acide hydroxy-4 méthoxy-3 mandélique :
  - . RR = 79,3 %.
  - . RT = 83,1 %

- acide hydroxy-2 méthoxy-3 mandélique :

  . RR = 5,6%
  . RT = 5,8 %

- acide hydroxy-2 méthoxy-3 dimandélique-1,5 :

  . RR = 8,0 %
  . RT = 4,2 %

Exemples 4 à 8

Dans la série d'exemples qui suit, on reproduit l'exemple 1 mais l'on met en oeuvre d'autres types d'acides dicarboxyliques tels que l'acide, malonique, succinique ainsi que l'E.D.T.A.
La solution d'acide glyoxylique mise en jeu contient de l'acide oxalique à raison de 0,09 %, des acides carboxyliques inférieurs tels que l'acide acétique à raison de 1 %, de l'acide formique et glycolique en une quantité respective inférieure à 0,3 %.
Toutes les conditions des exemples et les résultats obtenus sont consignés dans le tableau 1.

Tableau 1

| Ref. ex. | acide dicarboxylique* (%) | TT | RR | | | RT | | |
|---|---|---|---|---|---|---|---|---|
| | | | para | ortho | di | para | ortho | di |
| 4 | - | 45,2 | 77.5 | 4,9 | 7,6 | 84,3 | 5,3 | 4,1 |
| 5 | acide oxalique (2,0 %) | 47,8 | 80,1 | 4,8 | 8,1 | 83,0 | 5,0 | 4,2 |
| 6 | acide malonique (2,0 %) | 46,1 | 80,2 | 5,2 | 7,6 | 84,8 | 5,5 | 4,1 |
| 7 | acide succinique (1.9 %) | 48,0 | 81,4 | 5,6 | 8,0 | 85,0 | 5,8 | 4,2 |
| 8 | E.D.T.A. (1,5 %) | 44,5 | 80,6 | 4,9 | 7,7 | 88,0 | 5,4 | 4,2 |

* = acide dicarboxylique exprimé en % molaire par rapport à l'acide glyoxylique.

Exemples 9 à 11

Dans les exemples suivants, on augmente la quantité d'acide oxalique mis en oeuvre dans la solution d'acide glyoxylique.
On suit le protocole opératoire de l'exemple 1 et l'on met en oeuvre une solution d'acide glyoxylique à 50 % dont la composition est donnée dans les l'exemples 4 à 8.
Les résultats obtenus sont consignés dans le tableau suivant :

Tableau II

| Ref. ex. | acide oxalique * | TT | RR | | | RT | | |
|---|---|---|---|---|---|---|---|---|
| | | | para | ortho | di | para | ortho | di |
| 9 | 1,00 | 45,2 | 79,5 | 5,1 | 7,6. | 86,7 | 5,6 | 4,1 |
| 10 | 1.29 | 47,8 | 80,1 | 4,8 | 8,1 | 83,0 | 5.0 | 4,2 |
| 11 | 1,78 | 46,1 | 76,5 | 4,8 | 8,0 | 83,0 | 5,2 | 4,3 |

* = concentration d'acide oxalique en % poids dans la solution d'acide glyoxylique.

Dans ledit tableau, les abréviations ortho, para et di signifient :

- acide hydroxy-4 méthoxy-3 mandélique = para
- acide hydroxy-2 méthoxy-3 mandélique = ortho
- acidé hydroxy-2 méthoxy-3 dimandélique-1,5 = di

**Revendications**

1. Procédé de préparation de composés p-hydroxymandéliques éventuellement substitués et dérivés qui consiste à effectuer la condensation dans l'eau, en présence d'un agent alcalin, d'un composé aromatique porteur d'au moins un groupe hydroxyle et dont la position en para est libre, avec l'acide glyoxylique, ledit procédé étant **caractérisé par le fait que** la réaction est conduite en présence d'une quantité efficace d'un composé porteur d'au moins deux fonctions carboxyliques.

2. Procédé selon la revendication 1 **caractérisé par le fait que** le composé aromatique hvdroxylé répond à la formule suivante (I) :

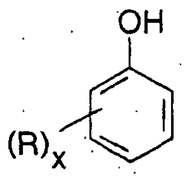

(I)

dans ladite formule (I) :

- la position en para est libre,
- x est un nombre entier compris entre 1 et 4,
- R représente :
  . un atome d'hydrogène,
  . un groupe hydrocarboné ayant de 1 à 20 atomes de carbone choisi parmi les groupes alkyle, alkoxy, hydroxyalkyle, cycloalkyle, aryle, phénoxy, alkoxyalkyle, fluoroalkyle, hydroxyalkoxyalkylène,
  . un groupe hydroxyle,
  . un groupe -CHO,
  . un groupe acyle ayant de 2 à 6 atomes de carbone,
  . un atome d'halogène, de préférence un atome de fluor, de chlore ou de . brome.
  . deux groupes R placés sur deux atomes de carbone vicinaux peuvent former ensemble et avec les atomes de carbone qui les portent un cycle benzénique.

3. Procédé selon l'une des revendications 1 et 2 **caractérisé par le fait que** le composé aromatique hydroxylé répond à la formule (I) dans laquelle :

- x est égal à 0, 1, 2 ou 3,
- représente l'un des groupes ou fonctions suivantes :
  . un atome d'hydrogène,
  . un radical alkyle, linéaire ou ramifié, ayant de 1 à 10 atomes de carbone, de préférence de 1 à 4 atomes de carbone,
  . un radical alkoxy linéaire ou ramifié ayant de 1 à 10 atomes de carbone, de préférence de 1 à 4 atomes de carbone,
  . un groupe - OH,
  . un groupe - CHO,
  . un atome d'halogène.
  . un groupe - $CF_3$

4. Procédé selon l'une des revendeications 1 à 3 **caractérisé par le fait que** le composé aromatique hydroxylé répond à la formule (I) dans laquelle les radicaux R identiques ou différents sont un atome d'hydrogène, un radical alkyle, linéaire ou ramifié ayant de 1 à 4 atomes de carbone, un radical alkoxy linéaire ou ramifié ayant de 1 à 4 atomes de carbone, un groupe - CHO, un atome de chlore et x est de préférence égal à 0 ou 1.

5. Procédé selon l'une des revendications 1 à 4 **caractérisé par le fait que** le composé aromatique hydroxylé de formule (I) est le phénol, l'o-crésol, le m-crésol, l'éthyl-3 phénol, le tert-butyl-2 phénol, le gaïacol, le guétol, l'isopropoxy-2 phénol.

6. Procédé selon l'une des revendications 1 à 5 **caractérisé par le fait que** le catalyseur est un composé porteur d'au moins deux fonctions carboxyliques répondant à la formule suivante (II) :

$$HOOC - R_1 - COOH \qquad (II)$$

dans ladite formule (II), $R_1$ représente un lien valentiel ou un radical hydrocarboné éventuellement substitué comportant de 1 à 40 atomes de carbone.

7. Procédé selon la revendication 6 **caractérisé par le fait que** le catalyseur est un composé porteur d'au moins deux fonctions carboxyliques répondant à la formule (II) dans laquelle $R_1$ symbolise un radical hydrocarboné, substitué ou non qui peut être un radical aliphatique acyclique, saturé ou insaturé, linéaire ou ramifié ; un radical carbocyclique, saturé, insaturé ou aromatique, monocyclique ou polycyclique ; un radical hétérocyclique, saturé, insaturé ou aromatique, monocyclique ou polycyclique.

8. Procédé selon la revendication 6 **caractérisé par le fait que** lé catalyseur est un composé porteur d'au moins deux fonctions carboxyliques répondant à la formule (II) dans laquelle $R_1$ représente un reste aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons; la chaîne hydrocarbonée pouvant être éventuellement :

(1) - interrompue par l'un des groupés suivants dénommés Y :

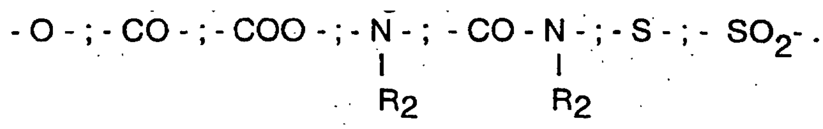

dans ces formules $R_2$ représente l'hydrogène ou un radical alkyle linéaire ou ramifié ayant de 1 à 4 atomes de carbone, ou un radical de type $-(CH_2)_p - COOH$ dans lequel p est un nombre compris entre 1 et 5,
(2) - et/ou porteuse de l'un des substituants suivants :

$-OH; -COOH; -CHO; -NO_2; -CN; -NH_2; -SH; -X; -CF_3.$
$- NH - [(CH_2)_p - COOH]$ ou $- N - [(CH_2)_p - COOH]_2$

avec X représentant un atome d'halogène, de préférence un atome de fluor, de chlore ou de brome et p ayant la signification donnée précédemment.

9. Procédé selon la revendication 6 **caractérisé par le fait que** le catalyseur est un composé porteur d'au moins deux fonctions carboxyliques répondant à la formule (II) dans laquelle $R_1$ représente un reste hydrocarboné aromatique, et notamment benzénique répondant à la formule générale (III) :

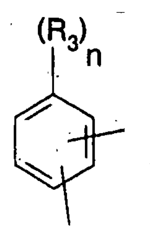

(III)

dans ladite formule (III) :

- n est un nombre entier de 0 à 4, de préférence de 0 à 3,
- $R_3$ représente l'un des groupes ou fonctions suivantes :

- . un atome d'hydrogène,
- . un radical alkyle linéaire ou ramifié, ayant de 1 à 4 atomes de carbone,
- . un radical alkoxy linéaire ou ramifié ayant de à 4 atomes de carbone,
- . un radical méthylène ou éthylène dioxy,
- . un groupe - CHO,
- . un radical phényle ou benzyle,
- . un atome d'halogène.

**10.** Procédé selon la revendication 6 **caractérisé par le fait que** le catalyseur est un composé porteur d'au moins deux fonctions carboxyliques répondant à la formule (II) dans laquelle le radical $R_1$ représente un reste divalent hydrocarboné aromatique polycyclique ; les cycles pouvant former entre eux des systèmes ortho- condensés, ortho- et péri-condensés.

**11.** Procédé selon la revendication 6 **caractérisé par le fait que** le catalyseur est un composé porteur d'au moins deux fonctions carboxyliques répondant à la formule (II) dans laquelle, $R_1$ représente un reste carbocyclique saturé ou comprenant 1 ou 2 insaturations dans le cycle, ayant généralement de 3 à 7 atomes de carbone, de préférence, 6 atomes de carbone dans le cycle.

**12.** Procédé selon l'une des revendications 6 à 11 **caractérisé par le fait que** le catalyseur est un composé porteur d'au moins deux fonctions carboxyliques répondant à la formule (II) dans laquelle $R_1$ représente un radical divalent constitué par un enchaînement de deux à quatre restes tels que définis précédemment, reste aliphatique, reste aromatique ou cycloaliphatique, reliés entre eux par un lien valentiel ou par un groupe fonctionnel.

**13.** Procédé selon la revendication 6 **caractérisé par le fait que** le catalyseur est un composé porteur d'au moins deux fonctions carboxyliques répondant à la formule (II) choisi parmi :

- les acides aliphatiques dicarboxyliques tels que :

  - . l'acide oxalique
  - . l'acide malonique
  - . l'acide succinique
  - . l'acide glutarique
  - . l'acide adipique
  - . l'acide diméthyl-2,4 adipique
  - . l'acide pimélique
  - . l'acide subérique
  - . l'acide azélaïque
  - . l'acide sébacique
  - . l'acide dodécane dioïque
  - . l'acide fumarique
  - . l'acide maléique

- les acides cycloalcanedicarboxyliques tels que l'acide cyclohexane dicarboxylique-1,4,
- les acides aromatiques dicarboxyliques tels que :

  - . l'acide phtalique
  - . l'acide isophtalique
  - . l'acide téréphtalique
  - . l'acide phènylènediacétique
  - . l'acide naphtalène dicarboxylique-1,5
  - . l'acide naphtalène dicarboxylique-1,6
  - . l'acide diphénylcarboxylique-4,4'
  - . l'acide diphénylcarboxylique-3,3'
  - . l'oxyde de bis(hydroxycarbonyl-4) phényle
  - . l'oxyde de bis(hydroxycarbonyl-3)phényle
  - . la dihydroxycarbonyl-4,4' diphénylsulfone
  - . la dihydroxycarbonyl-3,3' diphénylsulfone

- les acides pyrimidines ou imidazoles dicarboxyliques.
- les acides aminopolycarboxyliques:

  . l'acide éthylènediaminotétracétique (E.D.T.A.)
  . l'acide diéthylènetriaminopentacétique (D.T.P.A.)
  . l'acide nitrilotriacétique (N.T.A.)
  . l'acide N-(2-hydroxyéthyl)éthylène diaminotriacétique (H.E.D.T.A.)

**14.** Procédé selon l'une des revendications 1 à 13 **caractérisé par le fait que** la solution aqueuse d'acide glyoxylique comprend des acides monofonctionnels, notamment de 0,1 à 3 % d'acide acétique.

**15.** Procédé selon l'une des revendications 1 à 14 **caractérisé par le fait que** la solution aqueuse d'acide glyoxylique à une concentration variant de 15 à 70 % en poids, de préférence aux environs de 50 % en poids.

**16.** Procédé selon l'une des revendications 1 à 15 **caractérisé par le fait que** le rapport molaire entre le composé aromatique hydroxylé de formule (I) et l'acide glyoxylique varie entre 1,5 et 4,0 et est choisi préférentiellement entre 2,0 et 3,0.

**17.** Procédé selon l'une des revendications 1 à 16 **caractérisé par le fait que** la quantié d'hydroxyde de métal alcalin est voisine ou égale de la quantité stoechiométrique nécessaire pour salifier tous les groupements salifiables du composé aromatique hydroxylé de formule (I) et pour salifier la fonction carboxylique de l'acide glyoxylique.

**18.** Procédé selon l'une des revendications 1 à 17 **caractérisé par le fait que** la concentration du composé aromatique hydroxylé de formule (I) est comprise de préférence entre 0,5 et 1,5 moles/litre, et plus particulièrement aux environs de 1 mole/litre.

**19.** Procédé selon l'une des revendications 1 à 18 **caractérisé par le fait que** la quantité de catalyseur mise en oeuvre est telle que le rapport molaire entre le catalyseur et le composé aromatique hydroxylé de formule (I) se situe entre 0,005 et 0,025 et de préférence entre 0,01 et 0,02.

**20.** Procédé selon l'une des revendications 1 à 19 **caractérisé par le fait que** la quantité de catalyseur mise en oeuvre exprimée par le rapport entre le nombre de moles de catalyseur et le nombre de moles d'acide glyoxylique est choisie entre 0,5 et 2,5 % de préférence, entre 1 et 2 %.

**21.** Procédé selon l'une des revendications 1 à 20 **caractérisé par le fait que** le catalyseur est apporté tout ou partie par la solution aqueuse d'acide glyoxylique.

**22.** Procédé selon la revendication 21 **caractérisé par le fait que** que la solution d'acide glyoxylique comprend de 0,6 à 3 %, de préférence, de 1,2 à 2,6 % en poids d'acide oxalique exprimé par rapport au poids d'acide glyoxylique.

**23.** Procédé selon l'une des revendications à 1 à 22 **caractérisé par le fait que** le catalyseur est introduit avec la solution aqueuse d'acide glyoxylique ou dans le milieu réactionnel de départ, comprenant le composé aromatique hydroxylé de formule (I), de l'eau et l'hydroxyde de métal alcalin.

**24.** Procédé selon l'une des revendications 1 à 23 **caractérisé par le fait que** la température de la réaction varie entre 20°C et 60°C, de préférence entre, 30°C et 40°C.

**25.** Procédé de préparation d'acides hydroxyarylacétiques **caractérisé par le fait que** l'on prépare un composé p-hydroxymandélique éventuellement substitué par un procédé selon l'une des revendications 1 à 24, et que l'on réduit ensuite ledit composé.

**26.** Procédé de préparation des acides hydroxyarylglyoxyliques ou des aldéhydes hydroxyaromatiques **caractérisé par le fait que** l'on prépare un composé p-hydroxymandélique éventuellement substitué par un procédé selon l'une des revendications 1 à 24, et que l'on oxyde ensuite ledit composé.

**27.** Procédé de préparation de l'hydroxy-4 benzaldéhyde **caractérisé par le fait que** l'on prépare l'acide p-hydroxymandélique par un procédé selon l'une des revendications 1 à 24, et que l'on oxyde ensuite ledit composé.

**28.** Procédé de préparation de la vanilline **caractérisé par le fait que** l'on prépare l'acide méthoxy-3 p-hydroxymandélique par un procédé selon l'une des revendications 1 à 24, et que l'on oxyde ensuite ledit composé.

**29.** Procédé de préparation de l'éthyl-3 vanilline **caractérisé par le fait que** l'on prépare l'acide éthoxy-3 p-hydroxymandélique par un procédé selon l'une des revendications 1 à 24, et que l'on oxyde ensuite ledit composé.

**30.** Procédé de préparation de l'isopropyl-3 vanilline **caractérisé par le fait que** l'on prépare l'acide isopropoxy-3 p-hydroxymandétique par un procédé selon l'une des revendications 1 à 24, et que l'on oxyde ensuite ledit composé.

**Claims**

**1.** A process for the preparation of optionally substituted p-hydroxymandelic compounds and derivatives, which consists in carrying out the condensation, in water, in the presence of an alkaline agent, of an aromatic carrier compound with at least one hydroxyl group and the para position of which is free, with glyoxylic acid, said process being **characterised in that** the reaction is carried out in the presence of an effective quantity of a carrier compound with at least two carboxylic functions.

**2.** A process according to claim 1, **characterised in that** the hydroxylated aromatic compound corresponds to the following formula (I):

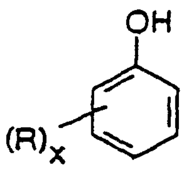
(I)

in which formula (I):

- the para position is free,
- x is an integer between 1 and 4,
- R represents :
  - a hydrogen atom,
  - a hydrocarbon group having from 1 to 20 carbon atoms selected from the alkyl, alkoxy, hydroxyalkyl, cycloalkyl, aryl, phenoxy, alkoxyalkyl, fluoroalkyl, hydroxyalkoxyalkylene groups,
  - a hydroxyl group,
  - a -CHO group,
  - an acyl group having from 2 to 6 carbon atoms,
  - a halogen atom, preferably a fluorine, chlorine or bromine atom,
  - two R groups placed on two vicinal carbon atoms can form together and with the carbon atoms which carry them a benzene ring.

**3.** A process according to either claim 1 or claim 2, **characterised in that** the hydroxylated aromatic compound corresponds to formula (I), in which:

- x is equal to 0, 1, 2 or 3,
- R represents one of the following groups or functions:
  - . a hydrogen atom,
  - . a linear or branched alkyl radical having from I to 10 carbon atoms, and preferably from 1 to 4 carbon atoms,
  - . a linear or branched alkoxy radical having from 1 to 10 carbon atoms, preferably from 1 to 4 carbon atoms,
  - . an -OH group,
  - . a -CHO group,
  - . a halogen atom,

. a $-CF_3$ group.

**4.** A process according to one of claims 1 to 3, **characterised in that** the hydroxylated aromatic compound corresponds to formula (I) in which the R radicals which are identical or different are a hydrogen atom, a linear or branched alkyl radical with 1 to 4 carbon atoms, a linear or branched alkoxy radical with 1 to 4 carbon atoms, a -CHO group, a chlorine atom, and x is preferably equal to 0 or 1.

**5.** A process according to one of claims I to 4, **characterised in that** the hydroxylated aromatic compound of formula (I) is phenol, o-cresol, m-cresol, 3-ethyl phenol, 2-tert-butyl phenol, guaiacol, guetol, 2-isopropoxy phenol.

**6.** A process according to one of claims 1 to 5, **characterised in that** the catalyst is a compound carrying at least two carboxylic functions corresponding to the following formula (II):

$$HOOC\text{-}R_t\text{-}COOH \qquad (II)$$

in which formula (II), $R_1$ represents a valency bond or an optionally substituted hydrocarbon radical containing 1 to 40 carbon atoms.

**7.** A process according to claim 6, **characterised in that** the catalyst is a carrier compound having at least two carboxylic functions corresponding to formula (II) wherein $R_1$ symbolises a substituted or non-substituted hydrocarbon radical which can be a linear or branched, saturated or unsaturated acyclic aliphatic radical; a monocyclic or polycyclic, saturated, unsaturated, or aromatic carbocyclic radical; a monocyclic or polycyclic, saturated, unsaturated or aromatic heterocyclic radical.

**8.** A process according to claim 6, **characterised in that** the catalyst is a carrier compound with at least two carboxylic functions corresponding to formula (II), in which $R_1$ represents a linear or branched, acyclic aliphatic residue having preferably 1 to 12 carbon atoms, saturated or containing one or more unsaturations on the chain, generally 1 to 3 unsaturations which can be single or conjugated double bonds, or triple bonds; the hydrocarbon chain can optionally be:

(1) - interrupted by one of the following groups called Y:

$$-O-;\ -CO-;\ -COO-;\ -\underset{\displaystyle R_2}{\underset{|}{N}}-;\ -CO-\underset{\displaystyle R_2}{\underset{|}{N}};\ -S-;\ -SO_2-$$

in which formulae $R_2$ represents hydrogen or a linear or branched alkyl radical having 1 to 4 carbon atoms, or a radical of $-(CH_2)_p$ - COOH type in which p is a number between 1 and 5,

(2) - and/or bearing one of the following substituents:

- OH; - COOH ; - CHO ; - $NO_2$; - CN; $-NH_2$; - SH; -X ; $-CF_3$
- NH - $[(CH_2)_P$ - COOH] or - N - $[(CH_2)_p$ - $COOH]_2$

with X representing a halogen atom, preferably a fluorine, chlorine or bromine atom, and p having the meaning given hereinabove.

**9.** A process according to claim 6, **characterised in that** the catalyst is a carrier compound with at least two carboxylic functions corresponding to formula (II), in which $R_1$ represents an aromatic hydrocarbon residue, and, in particular, a benzene residue corresponding to the general formula (III):

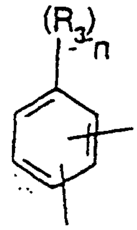

(III)

in which formula (III):

- n is an integer from 0 to 4, preferably from 0 to 3,
- $R_3$ represents one of the following groups or functions,
  - . a hydrogen atom,
  - . a linear or branched alkyl radical having from 1 to 4 carbon atoms,
  - . a linear or branched alkoxy radical having from 1 to 4 carbon atoms,
  - . a methylene or ethylene dioxy radical,
  - . a -CHO group,
  - . a phenyl or benzyl radical,
  - . a halogen atom.

**10.** A process according to claim 6, **characterised in that** the catalyst is a carrier compound with at least two carboxylic functions corresponding to formula (II) in which the $R_1$ radical represents a polycyclic aromatic hydrocarbon divalent residue; the rings can form between themselves ortho-condensed, ortho- and peri-condensed systems.

**11.** A process according to claim 6, **characterised in that** the catalyst is a carrier compound with at least two carboxylic functions corresponding to formula (II), in which $R_1$ represents a carbocyclic residue which is saturated or contains 1 or 2 unsaturations in the ring, generally having 3 to 7 carbon atoms, preferably 6 carbon atoms, in the ring.

**12.** A process according to one of claims 6 to 11, **characterised in that** the catalyst is a carrier compound with at least two carboxylic functions corresponding to formula (II), in which $R_1$ represents a divalent radical constituted by a chain formation of two to four residues as defined hereinabove, an aliphatic residue, an aromatic residue or a cycloaliphatic residue, connected together by a valency bond or by a function group.

**13.** A process according to claim 6, **characterised in that** the catalyst is a carrier compound with at least two carboxylic functions corresponding to formula (II) selected from:

- dicarboxylic aliphatic acids, such as:
  - . oxalic acid
  - . malonic acid
  - . succinic acid
  - . glutaric acid
  - . adipic acid
  - . 2,4-dimethyl adipic acid
  - . pimelic acid
  - . suberic acid
  - . azelaic acid
  - . sebacic acid
  - . dodecane dioic acid
  - . fumaric acid maleic acid
- cycloalkanedicarboxylic acids, such as cyclohexane 1,4-dicarboxylic acid,
- aromatic dicarboxylic acids, such as:

- phthalic acid
- isophthalic acid
- terephthalic acid
- phenylenediacetic acid
- naphthalene 1,5-dicarboxylic acid
- naphthalene 1,6-dicarboxylic acid
- 4,4'-diphenylcarboxylic acid
- 3,3'-diphenylcarboxylic acid
- bis(4-hydroxycarbonyl) phenyl oxide
- bis(3-hydroxycarbonyl) phenyl oxide
- 4,4'-dihydroxycarbonyl diphenylsulphone
- 3,3'-dihydroxycarbonyl diphenylsulphone

- pyrimidine or imidazole dicarboxylic acids.
- aminopolycarboxylic acids:

  - ethylenediaminotetracetic acid (E.D.T.A.)
  - diethytenetriaminopentacetic acid (D.T.P.A.)
  - nitrilotriacetic acid (N.T.A.)
  - N-(2-hydroxyethyl)ethylene diaminotriacetic acid (H.E.D.T.A.).

**14.** A process according to one of claims I to 13, **characterised in that** the aqueous solution of glyoxylic acid contains monofunctional acids, in particular from 0.1 to 3% acetic acid.

**15.** A process according to one of claims 1 to 14, **characterised in that** the aqueous solution of glyoxylic acid has a concentration which varies from 15 to 70% by weight, preferably in the region of 50% by weight.

**16.** A process according to one of claims 1 to 15, **characterised in that** the molar ratio between the hydroxylated aromatic compound of formula (I) and the glyoxylic acid varies between 1.5 and 4.0 and is preferably selected between 2.0 and 3.0.

**17.** A process according to one of claims 1 to 16, **characterised in that** the quantity of alkali metal hydroxide is in the region of, or equal to, the stoichiometric quantity necessary to salify all the salifiable groups of the hydroxylated aromatic compound of formula (I) and to salify the carboxylic function of the glyoxylic acid.

**18.** A process according to one of claims 1 to 17, **characterised in that** the concentration of the hydroxylated aromatic compound of formula (I) is preferably comprised between 0.5 and 1.5 moles/litre, and, more particularly, in the region of 1 mole/litre.

**19.** A process according to one of claims 1 to 18, **characterised in that** the quantity of catalyst used is such that the molar ratio between the catalyst and the hydroxylated aromatic compound of formula (I) is between 0.005 and 0.025, and preferably between 0.01 and 0.02.

**20.** A process according to one of claims 1 to 19, **characterised in that** the amount of catalyst used, expressed by the ratio between the number of moles of catalyst and the number of moles of glyoxylic acid is selected between 0.5 and 2.5%, preferably between 1 and 2%.

**21.** A process according to one of claims 1 to 20, **characterised in that** the catalyst is introduced completely or partially with the aqueous solution of glyoxylic acid.

**22.** A process according to Claim 21, **characterised in that** the solution of glyoxylic acid comprises from 0.6 to 3%, preferably from 1.2 to 2.6% by weight of oxalic acid, expressed in relation to the weight of glyoxylic acid.

**23.** A process according to one of Claims 1 to 22, **characterised in that** the catalyst is introduced with the aqueous solution of glyoxylic acid, or into the starting reaction medium comprising the hydroxylated aromatic compound of formula (I), water and the hydroxide of the alkaline metal.

**24.** A process according to one of Claims 1 to 23, **characterised in that** the reaction temperature varies between

20°C and 60°C, preferably between 30°C and 40°C.

**25.** A process for the preparation of hydroxyarylacetic acids, **characterised in that** an optionally substituted p-hydroxymandelic compound is prepared by way of a process according to one of Claims 1 to 24, and **in that** said compound is then reduced.

**26.** A process for the preparation of hydroxyarylglyoxylic acids or hydroxyaromatic aldehydes, **characterised in that** an optionally substituted p-hydroxymandelic compound is prepared by way of a process according to one of Claims 1 to 24, and **in that** said compound is then oxidised.

**27.** A process for the preparation of 4-hydroxy benzaldehyde, **characterised in that** p-hydroxymandelic acid is prepared by way of a process according to one of Claims 1 to 24, and **in that** said compound is then oxidised.

**28.** A process for the preparation of vanilline, **characterised in that** 3-methoxy p-hydroxymandelic acid is prepared by way of a process according to one of Claims 1 to 24, and **in that** said compound is then oxidised.

**29.** A process for the preparation of 3-ethyl vanilline, **characterised in that** 3-ethoxy p-hydroxy mandelic acid is prepared by way of a process according to one of Claims 1 to 24, and **in that** said compound is then oxidised.

**30.** A process for the preparation of 3-isopropyl vanilline, **characterised in that** 3-isopropoxy p-hydroxymandelic acid is prepared by way of a process according to one of Claims 1 to 24, and **in that** said compound is then oxidised.

**Patentansprüche**

**1.** Verfahren zur Herstellung von gegebenenfalls substituierten p-Hydroxymandelsäureverbindungen und ihren Derivaten, wobei eine Kondensation einer aromatischen Verbindung mit mindestens einer Hydroxylgruppe, deren para-Position frei ist, mit Glyoxylsäure in Wasser in Gegenwart eines alkalischen Reagenzes durchgeführt wird, **dadurch gekennzeichnet, daß** die Reaktion in Gegenwart einer wirksamen Menge einer Verbindung mit mindestens zwei Carboxylfunktionen durchgeführt wird.

**2.** Verfahren nach Anspruch 1, **dadurch gekennzeichnet, daß** die hydroxylierte aromatische Verbindung der folgenden Formel (I) entspricht:

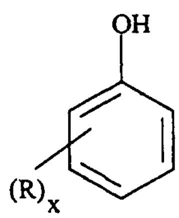

(I)

wobei in der Formel (I):

- die para-Position frei ist,
- x eine ganze Zahl zwischen 1 und 4 ist,
- R darstellt:
    - . ein Wasserstoffatom,
    - . eine Kohlenwasserstoffgruppe mit 1 bis 20 Kohlenstoffatomen, ausgewählt aus Alkyl-, Alkoxy-, Hydroxyalkyl-, Cycloalkyl-, Aryl-, Phenoxy-, Alkoxyalkyl-, Fluoralkyl- und Hydroxyalkoxyalkylengruppen,
    - . eine Hydroxylgruppe,

- . eine Gruppe -CHO,
- . eine Acylgruppe mit 2 bis 6 Kohlenstoffatomen,
- . ein Halogenatom, vorzugsweise ein Fluor-, Chlor- oder Bromatom,

wobei zwei an zwei vicinalen Kohlenstoffatomen befindliche Gruppen R zusammen und mit den Kohlenstoffatomen, von denen sie getragen werden, einen Benzolring bilden können.

**3.** Verfahren nach Anspruch 1 oder 2, **dadurch gekennzeichnet, daß** die hydroxylierte aromatische Verbindung der Formel (I) entspricht, wobei:

- x gleich 0, 1, 2 oder 3 ist,
- R eine der folgenden Gruppen oder Funktionen darstellt:
  - . ein Wasserstoffatom,
  - . einen linearen oder verzweigten Alkylrest mit 1 bis 10 Kohlenstoffatomen, vorzugsweise mit 1 bis 4 Kohlenstoffatomen,
  - . einen linearen oder verzweigten Alkoxyrest mit 1 bis 10 Kohlenstoffatomen, vorzugsweise 1 bis 4 Kohlenstoffatomen,
  - . eine Gruppe -OH,
  - . eine Gruppe -CHO,
  - . ein Halogenatom,
  - . eine Gruppe $-CF_3$.

**4.** Verfahren nach einem der Ansprüche 1 bis 3, **dadurch gekennzeichnet, daß** die hydroxylierte aromatische Verbindung der Formel (I) entspricht, wobei die Reste R, identisch oder verschieden, ein Wasserstoffatom, ein linearer oder verzweigter Alkylrest mit 1 bis 4 Kohlenstoffatomen, ein linearer oder verzweigter Alkoxyrest mit 1 bis 4 Kohlenstoffatomen, eine Gruppe -CHO oder ein Chloratom sind und x vorzugsweise gleich 0 oder 1 ist.

**5.** Verfahren nach einem der Ansprüche 1 bis 4, **dadurch gekennzeichnet, daß** die hydroxylierte aromatische Verbindung der Formel (I) Phenol, o-Kresol, m-Kresol, 3-Ethylphenol, tert.-2-Butylphenol, Guajakol, Guetol oder 2-Isopropoxyphenol ist.

**6.** Verfahren nach einem der Ansprüche 1 bis 5, **dadurch gekennzeichnet, daß** der Katalysator eine Verbindung mit mindestens zwei Carboxylfunktionen entsprechend der folgenden Formel (II) ist:

$$HOOC\text{-}R_1\text{-}COOH \qquad (II)$$

wobei in der genannten Formel (II) $R_1$ eine Valenzbindung oder einen gegebenenfalls substituierten Kohlenwasserstoffrest mit 1 bis 40 Kohlenstoffatomen darstellt.

**7.** Verfahren nach Anspruch 6, **dadurch gekennzeichnet, daß** der Katalysator eine Verbindung mit mindestens zwei Carboxylfunktionen entsprechend der Formel (II) ist, wobei R, darstellt: einen gegebenenfalls substituierten Kohlenwasserstoffrest, der ein linearer oder verzweigter, gesättigter oder ungesättigter, acyclischer aliphatischer Rest sein kann; einen monocyclischen oder polycyclischen, gesättigten, ungesättigten oder aromatischen carbocyclischen Rest; einen monocyclischen oder polycyclischen, gesättigten, ungesättigten oder aromatischen heterocyclischen Rest.

**8.** Verfahren nach Anspruch 6, **dadurch gekennzeichnet, daß** der Katalysator eine Verbindung mit mindestens zwei Carboxylfunktionen entsprechend der Formel (II) ist, wobei $R_1$ darstellt: einen linearen oder verzweigten, acyclischen aliphatischen Rest mit vorzugsweise 1 bis 12 Kohlenstoffatomen, der gesättigt ist oder einen oder mehrere ungesättigte Bindungen in der Kette aufweist, insbesondere 1 bis 3 ungesättigte Bindungen, die einfache oder konjugierte Doppelbindungen oder Dreifachbindungen sein können, wobei die Kohlenwasserstoffkette gegebenenfalls:

(1) - unterbrochen sein kann durch eine der nachfolgenden, als Y bezeichneten Gruppen:

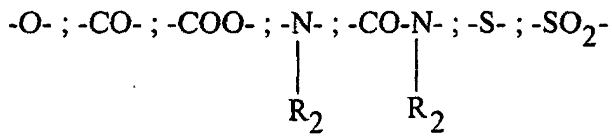

wobei in diesen Formeln $R_2$ darstellt: Wasserstoff oder einen linearen oder verzweigten Alkylrest mit 1 bis 4 Kohlenstoffatomen oder einen Rest vom Typ $-(CH_2)_p$ COOH, wobei p eine Zahl zwischen 1 und 5 ist,

(2) - und/oder Träger einer der folgenden Substituenten sein kann:

- OH ; -COOH ; -CHO ; $-NO_2$ ; -CN ; $-NH_2$ ; -SH ; -X ; $-CF_3$ ;
- $NH-[(CH_2)_p-COOH]$ oder $-N-[(CH_2)_p-COOH]_2$

wobei X ein Halogenatom, vorzugsweise ein Fluor-, Chlor- oder Bromatom darstellt und p die zuvor angegebenen Bedeutung hat.

**9.** Verfahren nach Anspruch 6, **dadurch gekennzeichnet, daß** der Katalysator einer Verbindung mit mindestens zwei Carboxylfunktionen entsprechend der Formel (II) ist, wobei $R_1$ einen aromatischen Kohlenwasserstoffrest und insbesondere einen Benzolrest entsprechend der allgemeinen Formel (III) darstellt:

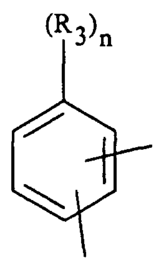

(III)

wobei in der Formel (III):

- n eine ganze Zahl von 0 bis 4, vorzugsweise von 0 bis 3, ist,
- $R_3$ eine der folgenden Gruppen oder Funktionen darstellt:
  - . ein Wasserstoffatom,
  - . einen linearen oder verzweigten Alkylrest mit 1 bis 4 Kohlenstoffatomen,
  - . einen linearen oder verzweigten Alkoxyrest mit 1 bis 4 Kohlenstoffatomen,
  - . einen Methylen- oder Dioxyethylenrest,
  - . eine Gruppe -CHO,

- einen Phenyl- oder Benzylrest
- ein Halogenatom.

**10.** Verfahren nach Anspruch 6, **dadurch gekennzeichnet, daß** der Katalysator eine Verbindung mit mindestens zwei Carboxylfunktionen entsprechend der Formel (II) ist, wobei der Rest $R_1$ einen polycyclischen, aromatischen divalenten (zweiwertigen) Kohlenwasserstoffrest darstellt, wobei die Ringe untereinander ortho-kondensierte oder ortho- und peri-kondensierte Systeme bilden können.

**11.** Verfahren nach Anspruch 6, **dadurch gekennzeichnet, daß** der Katalysator eine Verbindung mit mindestens zwei Carboxylfunktionen entsprechend der Formel (II) ist, wobei $R_1$ einen gesättigten oder 1 oder 2 ungesättigte Bindungen im Ring enthaltenden carbocyclischen Rest mit im allgemeinen 3 bis 7 Kohlenstoffatomen, vorzugsweise 6 Kohlenstoffatomen, im Ring darstellt.

**12.** Verfahren nach einem der Ansprüche 6 bis 11, **dadurch gekennzeichnet, daß** der Katalysator eine Verbindung mit mindestens zwei Carboxylfunktionen entsprechend der Formel (II) ist, wobei $R_1$ darstellt: einen divalenten (zweiwertigen) Rest aus einer Verkettung von zwei bis vier Resten, wie zuvor definiert, einen aliphatischen Rest, einen aromatischen oder cycloaliphatischen Rest, die untereinander durch eine Valenzbindung oder durch eine funktionelle Gruppe verbunden sind.

**13.** Verfahren nach Anspruch 6, **dadurch gekennzeichnet, daß** der Katalysator eine Verbindung mit mindestens zwei Carboxylfunktionen entsprechend der Formel (II) ist, ausgewählt aus:

- aliphatischen Dicarbonsäuren, wie
  - Oxalsäure
  - Malonsäure
  - Bernsteinsäure
  - Glutarsäure
  - Adipinsäure
  - 2,4-Dimethyladipinsäure
  - Pimelinsäure
  - Suberinsäure
  - Azelainsäure
  - Sebazinsäure
  - Dodecandisäure
  - Fumarsäure
  - Maleinsäure,
- Cycloalkandicarbonsäuren, wie 1,4-Dicarboxylcyclohexansäure,
- aromatischen Dicarbonsäuren, wie:
  - Phthalsäure
  - Isophthalsäure
  - Terephthalsäure
  - Phenylendiessigsäure

. 1,5-Dicarboxylnaphtalinsäure
. 1,6-Dicarboxylnaphtalinsäure
. 4,4'-Diphenylcarboxylsäure
. 3,3'-Diphenylcarboxylsäure
. Bis(4-hydroxycarbonyl)phenyloxid
. Bis(3-hydroxycarbonyl)phenyloxid
. 4,4'-Dihydroxycarbonyldiphenylsulfon
. 3,3'-Dihydroxycarbonyldiphenylsulfon,

- Pyrimidinsäuren oder Dicarboxylimidazolen,
- Aminopolycarboxylsäuren:

. Ehtylendiamintetraessigsäure (EDTA)
. Diethylentriaminpentaessigsäure (DTPA)
. Nitrilotriessigsäure (NTA)
. N-(2-Hydroxyethyl)ethylendiamintriessigsäure (HEDTA).

**14.** Verfahren nach einem der Ansprüche 1 bis 13, **dadurch gekennzeichnet, daß** die wäßrige Lösung der Glyoxylsäure monofunktionale Säuren, insbesondere 0,1 bis 3 % Essigsäure, aufweist.

**15.** Verfahren nach einem der Ansprüche 1 bis 14, **dadurch gekennzeichnet, daß** die wäßrige Lösung der Glyoxylsäure eine Konzentration von 15 bis 70 Gew.-%, vorzugsweise von etwa 50 Gew.-%, aufweist.

**16.** Verfahren nach einem der Ansprüche 1 bis 15, **dadurch gekennzeichnet, daß** das Molverhältnis zwischen der hydroxylierten aromatischen Verbindung der Formel (I) und der Glyoxylsäure zwischen 1,5 und 4,0 variiert und vorzugsweise zwischen 2,0 und 3,0 ausgewählt ist.

**17.** Verfahren nach einem der Ansprüche 1 bis 16, **dadurch gekennzeichnet, daß** die Menge des Alkalimetallhydroxids etwa oder gleich der stöchiometrischen Menge ist, die notwendig ist, um sämtliche versalzbaren Gruppen der hydroxylierten aromatischen Verbindung der Formel (I) zu versalzen und um die Carboxylfunktion der Glyoxylsäure zu versalzen.

**18.** Verfahren nach einem der Ansprüche 1 bis 17, **dadurch gekennzeichnet, daß** die Konzentration der hydroxylierten aromatischen Verbindung der Formel (I) vorzugsweise zwischen 0,5 und 1,5 mol/l liegt und besonders bevorzugt etwa 1 mol/l beträgt.

**19.** Verfahren nach einem der Ansprüche 1 bis 18, **dadurch gekennzeichnet, daß** die Menge des verwendeten Katalysators derart ist, daß das Molverhältnis zwischen dem Katalysator und der hydroxylierten aromatischen Verbindung der Formel (I) zwischen 0,005 und 0,025, vorzugsweise zwischen 0,01 und 0,02, beträgt.

**20.** Verfahren nach einem der Ansprüche 1 bis 19, **dadurch gekennzeichnet, daß** die Menge des verwendeten Katalysators, berechnet als Verhältnis der Molanzahl des Katalysators und der Molanzahl der Glyoxylsäure, zwischen 0,5 und 2,5 %, vorzugsweise zwischen 1 und 2 %, liegt.

**21.** Verfahren nach einem der Ansprüche 1 bis 20, **dadurch gekennzeichnet, daß** der Katalysator ganz oder teilweise durch die wäßrige Lösung der Glyoxylsäure eingebracht wird.

**22.** Verfahren nach Anspruch 21, **dadurch gekennzeichnet, daß** die Lösung der Glyoxylsäure 0,6 bis 3 Gew.-%, vorzugsweise 1,2 bis 2,6 Gew.-%, Oxalsäure enthält, bezogen auf das Gewicht der Glyoxylsäure.

**23.** Verfahren nach einem der Ansprüche 1 bis 22, **dadurch gekennzeichnet, daß** der Katalysator mit der wäßrigen Lösung der Glyoxylsäure oder in das Ausgangsreaktionsmilieu, das die hydroxylierte aromatische Verbindung der Formel (I), Wasser und das Alkalimetallhydroxid enthält, zugegeben wird.

**24.** Verfahren nach einem der Ansprüche 1 bis 23, **dadurch gekennzeichnet, daß** die Reaktionstemperatur zwischen 20 °C und 60 °C, vorzugsweise zwischen 30 °C und 40 °C, variiert.

**25.** Verfahren zur Herstellung von Hydroxyarylessigsäuren, **dadurch gekennzeichnet, daß** man eine gegebenenfalls

substituierte p-Hydroxymandelsäureverbindung nach einem Verfahren gemäß einem der Ansprüche 1 bis 24 herstellt und man anschließend diese Verbindung reduziert.

**26.** Verfahren zur Herstellung von Hydroxyarylglyoxylsäure oder hydroxyaromatischen Aldehyden, **dadurch gekennzeichnet, daß** man eine gegebenenfalls substituierte p-Hydroxymandelsäureverbindung nach einem Verfahren gemäß einem der Ansprüche 1 bis 24 herstellt und man anschließend diese Verbindung oxidiert.

**27.** Verfahren zur Herstellung von 4-Hydroxybenzaldehyd, **dadurch gekennzeichnet, daß** man p-Hydroxymandelsäure nach einem Verfahren gemäß einem der Ansprüche 1 bis 24 herstellt und man anschließend diese Verbindung oxidiert.

**28.** Verfahren zur Herstellung von Vanillin, **dadurch gekennzeichnet, daß** man 3-Methoxy-p-hydroxymandelsäure nach einem Verfahren gemäß einem der Ansprüche 1 bis 24 herstellt und man anschließend diese Verbindung oxidiert.

**29.** Verfahren zur Herstellung von 3-Ethylvanillin, **dadurch gekennzeichnet, daß** man 3-Ethoxy-p-hydroxymandelsäure nach einem Verfahren gemäß einem der Ansprüche 1 bis 24 herstellt und man anschließend diese Verbindung oxidiert.

**30.** Verfahren zur Herstellung von 3-Isopropylvanillin, **dadurch gekennzeichnet, daß** man 3-Isopropoxy-p-hydroxymandelsäure nach einem Verfahren gemäß einem der Ansprüche 1 bis 24 herstellt und man anschließend diese Verbindung oxidiert.